**Europäisches Patentamt**

**European Patent Office**

**Office européen des brevets**

(11) Numéro de publication: **0 224 420**
**B1**

(12) **FASCICULE DE BREVET EUROPEEN**

(45) Date de publication du fascicule du brevet:
21.03.90

(21) Numéro de dépôt: **86420257.7**

(22) Date de dépôt: **16.10.86**

(51) Int. Cl.⁴: **C07C 65/10**, C07C 51/02

(54) **Procédé de séparation et de purification de l'acide salicylique.**

(30) Priorité: **29.10.85 FR 8516259**

(43) Date de publication de la demande:
**03.06.87 Bulletin 87/23**

(45) Mention de la délivrance du brevet:
**21.03.90 Bulletin 90/12**

(84) Etats contractants désignés:
**AT BE CH DE ES GB GR IT LI LU NL SE**

(56) Documents cités:
**FR-A- 1 061 806**
**FR-A- 1 539 528**

(73) Titulaire: **RHONE-POULENC CHIMIE, 25, quai Paul Doumer, F-92408 Courbevoie Cédex(FR)**

(72) Inventeur: **Cocco, Roger, Rue de la Croix Rouge Solaize, F-69360 - Saint-Symphorien d'Ozon(FR)**

(74) Mandataire: **Vignally, Noel et al, RHONE-POULENC INTERSERVICES Service Brevets Chimie Centre de Recherches de Saint-Fons B.P. 62, F-69192 Saint-Fons Cedex(FR)**

## Description

La présente invention concerne la séparation et la purification de l'acide salicylique et, plus particulièrement, la précipitation de l'acide salicylique à partir de son sel de sodium et sa purification.

Le procédé de préparation de l'acide salicylique, le plus couramment utilisé, consiste dans la carboxylation à l'aide d'anhydride carbonique du phénate de sodium, sous une pression généralement supérieure à 50 bars et à une température de l'ordre de 150°C à 160°C.

On peut pour cette préparation se référer par exemple au brevet français No. 1 122 915.

Le phénate de sodium est de préférence mis en oeuvre en suspension dans du phénol libre, par exemple avec un rapport pondéral phénol/phénate de sodium de 3/1 à 5/1.

En fin de réaction la masse réactionnelle est traitée afin d'en séparer les sels de sodium des acides hydroxybenzoïques et hydroxyphtaliques obtenus, en l'occurrence essentiellement le salicylate monosodique, éventuellement le salicylate disodique et dans des proportions nettement moindres le parahydroxybenzoate de sodium et l'hydroxy-4 isophtalate de sodium.

Le salicylate de sodium obtenu par les procédés de carboxylation du phénate de sodium est généralement le salicylate monosodique (hydroxy-2 benzoate monosodique) contenant éventuellement du salicylate disodique. Dans ce que suit, sauf mention contraire, le terme salicylate de sodium concernera plus particulièrement le salicylate monosodique, contenant éventuellement du salicylate disodique, mais il reste bien entendu que le procédé de l'invention ne se limite pas au seul sel monosodique et peut tout aussie bien s'appliquer au salicylate disodique seul ou à des mélanges monosel/bisel contenant majoritairement du bisel.

Ce traitement peut consister notamment en une addition d'eau, qui dissout les sels de sodium des acides hydroxybenzoïques et hydroxyphtaliques, et en une extraction liquide-liquide à l'aide d'un solvant non miscible à l'eau. Une telle opération conduit ainsi à une solution organique contenant en particulier le phénol libre et une solution aqueuse contenant les sels de sodium indiqués précédemment.

Le procédé selon l'invention a pour but d'apporter un perfectionnement à la précipitation et à la purification de l'acide salicylique à partir de telles solutions aqueuses.

En effet généralement le traitement des solutions aqueuses de salicylate de sodium consiste à précipiter l'acide salicylique et les autres acides organiques par un acide minéral fort et notamment par de l'acide sulfurique. Ce traitement nécessite un fort excès d'acide minéral fort et plusieurs lavages successifs à l'eau pour éliminer le maximum de sel formé (sulfate de sodium le plus souvent). Ensuite l'acide salicylique obtenu peut être cristallisé une ou plusieurs fois pour diminuer le taux des autres acides organiques qu'il contient.

L'acide salicylique obtenu par le procédé habituel contient des quantités de sulfate de sodium, et à un moindre degré d'acide parahydroxybenzoïque et d'acide hydroxy-4 isophtalique, qui sont encore excessives pour certaines applications alimentaires ou médicales de l'acide salicylique.

La présente invention se propose de pallier ces inconvénients. En effet, elle permet selon les besoins soit d'obtenir plus simplement un acide salicylique de pureté suffisante pour des usages courants, soit d'obtenir un acide salicylique de très grande pureté, avec également une mise en oeuvre plus simple que dans les procédés antérieurs. Elle consiste en un procédé de précipitation et de purification de l'acide salicylique à partir d'une solution aqueuse de salicylate de sodium, caractérisé par la succession suivante de phases :

1. addition à ladite solution aqueuse d'un solvant organique de l'acide salicylique, en quantité suffisante pour dissoudre l'acide salicylique correspondant au sel de sodium engagé ;
2. addition d'un acide minéral fort en quantité au moins égale à la stoechiométrie par rapport au salicylate de sodium ;
3. séparation d'une phase essentiellement organique contenant l'acide salicylique et d'une phase aqueuse contenant le sel minéral de sodium formé.

Comme acide minéral fort on peut utiliser tout acide capable de déplacer l'acide salicylique de son sel; on peut citer à titre d'exemples non limitatifs l'acide sulfurique, l'acide chlorhydrique, l'acide phosphorique. Pour des questions de prix ou de moindre corrosion, on préfère généralement utiliser l'acide sulfurique. Dans la description du procédé de l'invention, on se référera donc par commodité le plus souvent à l'acidification par l'acide sulfurique, mais sans que soit pour autant exclu l'emploi d'autres acides minéraux forts.

Les solvants organiques qui conviennent pour la mise en oeuvre du procédé selon l'invention sont les solvants dans lesquels l'acide salicylique est soluble tandis que le sel minéral de sodium n'est pas soluble ou très peu soluble.

Ils peuvent être choisis parmi les éthers aliphatiques, les éthers aliphatiques substitués, les cétones aliphatiques, les cétones aliphatiques halogénées, les aldéhydes aliphatiques et les alcools aliphatiques.

Pour des questions pratiques et économiques, il n'est pas souhaitable d'utiliser dans solvants organiques ayant un point d'ébullition très élevé, car lors de l'isolement de l'acide salicylique le solvant sera généralement éliminé par distillation et l'acide salicylique devra contenir en fin de purification le minimum possible de traces du solvant employé.

C'est ainsi que l'on préférera un solvant ayant un point d'ébullition inférieur ou égal à 120°C et, plus particulièrement encore, inférieur ou égal à 100°C.

A titre d'exemples de ces solvants on peut citer :
– des éthers aliphatiques tels que l'oxyde de diisopropyle; l'oxyde de méthyle et de tertiobutyle; l'oxyde d'éthyle et d'isopropyle; l'oxyde d'éthyle et de propyle; l'oxyde de butyle et d'éthyle; l'oxyde d'éthyle et d'isobutyle; l'oxyde d'éthyle et de tertiobutyle; l'oxyde de butyle et de méthyle; l'oxyde d'isobutyl et de méthyle; l'oxyde de méthyle et de pentyle; l'oxyde de diéthyle; l'oxyde de dipropyle; l'oxyde d'isopropyle et de propyle; l'oxyde d'éthyle et de propynyle-1; l'oxyde d'éthyle et de propynyle-2; l'oxyde d'éthynyle et de propyle; l'oxyde d'allyle et d'éthyle; l'oxyde d'allyle et d'isopropyle; l'oxyde d'isopropyle et de vinyle ou l'oxyde d'isobutyle et de vinyle;
– des éthers aliphatiques halogénés tels que l'oxyde de bromo-2 éthyle et d'éthyle; l'oxyde de chloro-2 éthyle et d'éthyle;
– des cétones aliphatiques telles que l'acétone; la butanone-2; la méthyl-3 butanone-2; la diméthyl-3,3 butanone-2; la pentanone-2 ou la pentanone-3;
– des cétones aliphatiques chlorées telles que la chloro-3 butanone-2 ou la chloro-1 propanone-2;
– des aldéhydes aliphatiques tels que le propanal; le butanal; le méthyl-2 butanal; le méthyl-3 butanal; le pentanal; le méthoxyéthanal ou l'éthoxyéthanal;
– des aldéhydes aliphatiques chlorés tels que le chloréthanal; le dichloroéthanal; le chloro-2 propanal ou le chloro-2 méthyl-2 propanal;
– des alcools aliphatiques tels que le méthanol; l'éthanol; l'isopropanol; le n-propanol, le n-butanol; l'isobutanol ou le tertiobutanol.

Parmi les solvants qui sont utilisés dans le procédé selon l'invention, on préfère plus particulièrement les éthers aliphatiques, les éthers aliphatiques chlorés, les cétones aliphatiques et les cétones aliphatiques chlorées.

La quantité de solvant organique utilisée peut varier très largement.

La limite inférieure est constituée par la solubilité de l'acide salicylique dans ledit solvant à la température à laquelle on opère.

La limite supérieure est généralement non critique, mais il est bien évident qu'il n'est pas intéressant au plan économique d'opérer à de trop faibles concentrations finales d'acide salicylique dans le solvant.

En outre lorsque le solvant organique utilisé est miscible à l'eau, il est nécessaire que la concentration finale d'acide salicylique dans ledit solvant soit suffisante, pour qu'il se produise une démixtion entre une couche aqueuse contenant le sel minéral de sodium formé et une couche essentiellement organique contenant l'acide salicylique.

Pour ces raisons, la quantité de solvant utilisée est telle que la concentration finale en acide salicylique dans ledit solvant est généralement au moins égale à 8 % en poids.

La température à laquelle est mis en oeuvre le procédé selon l'invention est peu critique. Elle se situe habituellement entre 10°C et la température d'ébullition du solvant organique utilisé.

Cependant dans la mesure du possible, il vaut mieux éviter des températures trop basses peuvent conduire notamment à une cristallisation du sel minéral de sodium ou des températures trop élevées auxquelles la solubilité de l'acide salicylique dans l'eau est augmentée.

C'est pourquoi on opère de préférence à des températures de 40°C à 80°C, sans que cela représente un impératif.

La quantité d'acide minéral fort et plus particulièrement d'acide sulfurique utilisée est généralement légèrement supérieure à la quantité théoriquement nécessaire pour déplacer l'acide salicylique et les acides parahydroxybenzoïque et hydroxy-4 isophtalique de leurs sels respectifs de sodium.

Cet excès, qui est souvent d'environ de 5% à 10% de la quantité stoechiométrique, permet une fin de réaction plus rapide.

L'acide sulphurique est introduit habituellement sous forme d'une solution aqueuse telle que celles qui sont disponibles dans le commerce ; le plus fréquemment on utilise des solutions aqueuses ayant une concentration de 60% à 98% en poids.

En pratique, le procédé selon la présente invention peut être réalisé de la manière suivante :

A la solution aqueuse, contenant le salicylate de sodium à une concentration de 20 à 45% en poids et, le cas échéant, les sels de sodium des autres acides, est ajouté un solvant organique tel que défini précédemment en quantité suffisante pour dissoudre l'acide salicylique correspondant au salicylate de sodium engagé.

Selon le cas on obtient une phase homogène ou deux phases liquides.

On ajoute alors sous agitation l'acide sulfurique en quantité représentant environ 105% à 110% de la théorie nécessaire (la théorie étant 0,5 mole de $H_2SO_4$ pur pour 1 mole de salicylate monosodique ou de parahydroxybenzoate de sodium et 1 mole de $H_2SO_4$ pour 1 mole de salicylate disodique ou pour 1 mole d'hydroxy-4 isophtalate de sodium).

On amène la température à la valeur désirée.

Lorsque la réaction est terminée, on obtient deux phases liquides :
– une phase aqueuse contenant pratiquement tout le sulfate de sodium formé et très peu d'acide salicylique;
– une phase essentiellement organique contenant la quasi-totalité de l'acide salicylique formé.

En effet :
– soit l'on a mis en œuvre un solvant non miscible à l'eau et qui par conséquent décante;
– soit l'on a utilisé un solvant miscible à l'eau et l'on observe une démixtion entre la phase aqueuse chargée de sulfate de sodium et la phase organique chargée d'acide salicylique.

On opère ensuite la séparation de ces deux phases par décantation. La phase aqueuse contient généralement plus de 20% en poids de sulfate de sodium (qui peut, si on le souhaite, être récupéré) et moins de 0,5% en poids d'acide salicylique (généralement moins de 0,1% en poids d'acide salicylique lorsque l'on utilise un des solvants organiques préférés).

La phase essentiellement organique est alors traitée de manière connue en soi pour séparer l'acide salicylique.

On peut par exemple, si les applications envisagées ne nécessitent pas une pureté très supérieure à celle obtenue pour les procédés courants de séparation de l'acide salicylique, procéder à une atomisation de ladite phase organique, c'est-à-dire à sa pulvérisation à travers une buse à une température permettant la vaporisation instantanée du solvant. Ce procédé est simple et conduit à un acide salicylique de pureté au moins comparable à celle que donnent les procédés antérieurs.

On peut également, et c'est généralement la variante préférée, procéder à une distillation du solvant organique, suivie d'un refroidissement et d'une cristallisation de l'acide salicylique par addition d'eau.

L'acide salicylique obtenu selon cette dernière variante présente un taux de sulfate de sodium très faible, généralement inférieur à 0,01% en poids et, lorsque l'on utilise un des solvants organiques préférés, inférieur à 0,002% en poids et en taux d'acide parahydroxybenzoïque et d'acide hydroxy-4 isophtalique généralement inférieur à 0,01% et, lorsque l'on utilise l'un des solvants organiques préférés, inférieur à 0,005% en poids.

Un tel acide salicylique peut être utilisé dans les applications alimentaires ou médicales les plus exigeantes.

En outre on note que les cristaux d'acide salicylique ainsi préparés ont une granulométrie quie peut être contrôlée lors de la cristallisation en agissant sur la température à laquelle cette opération est efectuée. On obtient des aiguilles plus épaisses et une densité apparente plus élevée, ce qui permet une nette amélioration de la coulabilité de l'acide salicylique.

Le procédé de l'invention peut être mis en oeuvre de manière discontinue ou de manière continue. Il peut facilement être réalisé dans le cadre des procédés habituels de préparation d'acide salicylique à partir du phénate de sodium.

Les exemples qui suivent illustrent la présente invention.

## EXEMPLE 1

Dans un réacteur en verre de 2,5 litres, équipé d'une agitation efficace, on charge 750 g d'une solution aqueuse à 39,7 % en poids de salicylate de sodium (1,87 mole) comportant des impuretés organiques (sels de sodium de l'acide parahydroxyisophtalique et de l'acide parahydroxybenzoïque, en quantité totale correspondant environ à 1% du poids du salicylate de sodium), puis 600 g d'acétone.

On agite et on rajoute en 30 minutes 141 g d'une solution aqueuse d'acide sulfurique à 71% en poids (1,02 mole de $H_2SO_4$).

On poursuit l'agitation pendant 30 minutes tout en montant la température à 55°C.

On laisse ensuite décanter pendant 30 minutes environ à 55°C ; on obtient deux phases:
– une phase aqueuse inférieure
– une phase organique supérieure.

On soutire la phase aqueuse, toujours vers 55°C.

Cette phase aqueuse contient:
– environ 338 g d'eau,
– environ 6 g d'acétone,
– 136,3 g de sulfate de sodium et d'acide sulfurique,
– environ 2 g d'acide salicylique.

La phase acétonique contient:
– 456 g d'acétone,
– 162 g d'eau,
– 256,0 g d'acide salicylique (quantité dosée),
– 2,4 g d'acide parahydroxybenzoïque,
– 0,4 g d'acide hydroxy-4 isophtalique,
– 0,2 g de sulfate de sodium.

La phase organique est traitée de la manière suivante:
– on distille l'acétone tout en maintenant le volume constant de la phase par addition progressive d'eau;
– on arrête la distillation lorsque la température du condenseur est à 98°C– 100°C.

On obtient ainsi une nouvelle phase aqueuse comportant des cristaux d'acide salicylique en suspension.

On refroidit cette suspension de 100°C à 85°C en deux heures, puis de 85°C à 40°C en une heure.

On filtre à 40°C l'acide salicylique qui a cristallisé. On lave par environ 400 g d'eau l'acide salicylique, puis on le sèche.

On obtient 248 g d'acide salicylique sous forme d'un solide blanc cristallisé, ayant une bonne coulabilité.

L'acide salicylique contient comme impuretés:
– environ 0,005% d'acide parahydroxybenzoïque,
– environ 0,001% d'acide hydroxy-4 isophtalique,
– environ 0,001% de sulfate de sodium.

Le rendement de l'isolement et de la purification de l'acide salicylique par rapport au salicylate de sodium engagé est d'environ 96%.

Le rendement peut encore être amélioré par traitement de la phase aqueuse.

## EXEMPLE 2

On opère comme indiqué dans l'exemple 1, mais avec des charges plus faibles dans un réacteur en verre de 500 cm3 et en utilisant de l'oxyde de diisopropyle comme solvant.

On charge:
– 126 g d'une solution aqueuse contenant 30 g de salicylate de sodium;
– 65,5 g d'oxyde de diisopropyle;
– 11,2 g d'acide sulfurique à 95%.

Après les différentes phases opératoires décrites dans l'exemple 1, on obtient:
– 109 g de phase aqueuse;
– 89,6 g de phase organique contenant 25,5 g d'acide salicylique. En traitant la phase organique comme indiqué dans l'exemple 1, on obtient 25,1 g d'acide salicylique contenant 0,0003% de sulfate de sodium (97% de rendement par rapport au salicylate de sodium engagé).

## EXEMPLE 3

On opère comme indiqué dans l'exemple 1, mais avec des charges plus faibles dans un réacteur de 500 cm3 et en utilisant de l'éthanol comme solvant.

On charge:
– 126 g d'une solution aqueuse contenant 30 g de salicylate de sodium;
– 65 g d'éthanol;
– 11 g d'acide sulfurique à 95%.

Après les différentes phases opératoires décrites dans l'exemple 1, on obtient:
– 41 g de phase aqueuse,
– 158,4 g de phase organique (qui contient plus d'eau que dans l'exemple 2) contenant 25,0 g d'acide salicylique et 1,8 g de sulfate de sodium. En traitant la phase organique comme indiqué dans l'exemple 1, on obtient 24,5 g d'acide salicylique contenant 0,01% de sulfate de sodium (94% de rendement par rapport au salicylate de sodium engagé).

Avec l'éthanol on observe une moins bonne séparation des phases aqueuse et organique d'où une plus grande quantité de sulfate de sodium dans l'acide salicylique obtenu.

## Revendications

1. Procédé de purification de l'acide salicylique à partir d'une solution aqueuse de salicylate de sodium, caractérisé par la succession suivante de phases:
1. addition à ladite solution aqueuse d'un solvant organique de l'acide salicylique, en quantité suffisante pour dissoudre l'acide salicylique correspondant au sel de sodium engagé;
2. addition d'acide minéral fort en quantité au moins égale à la stoechiométrie par rapport au salicylate de sodium;
3. séparation d'une phase essentiellement organique contenant l'acide salicylique et d'une phase aqueuse contenant le sel minéral de sodium formé, et son traitement de manière connue en soi pour séparer l'acide salicylique.
2. Procédé selon la revendication 1, caractérisé en ce que l'acide minéral fort utilisé est l'acide sulfurique.
3. Procédé selon l'une des revendications 1 ou 2, caractérisé en ce que le solvant organique utilisé est choisie parmi les éthers aliphatiques, les éthers aliphatiques substitués, les cétones aliphatiques, les cétones aliphatiques halogénées, les aldéhydes aliphatiques et les alcools aliphatiques.
4. Procédé selon l'une des revendications 1 à 3, caractérisé en ce que le solvant organique est choisi parmi les éthers aliphatiques, les éthers aliphatiques chlorés, les cétones aliphatiques et les cétones aliphatiques chlorées.
5. Procédé selon l'une des revendications 1 à 4, caractérisé en ce que le solvant organique possède un point d'ébullition inférieur ou égal à 120°C et de préférence inférieur ou égal à 100°C.
6. Procédé selon l'une des revendications 1 à 5, caractérisé en ce que le solvant organique est choisi parmi:
– des éthers aliphatiques tels que l'oxyde de diisopropyle; l'oxyde de méthyle et de tertiobutyle; l'oxyde d'éthyle et d'isopropyle; l'oxyde d'éthyle et de propyle; l'oxyde de butyle et d'éthyle; l'oxyde d'éthyle et d'isobutyle; l'oxyde d'éthyle et de tertiobutyle; l'oxyde de butyle et de méthyle; l'oxyde d'isobutyle et de méthyle; l'oxyde de méthyle et de pentyle; l'oxyde de diéthyle; l'oxyde de dipropyle; l'oxyde d'isopropyle et de propyle; l'oxyde d'éthyle et de propynyle-1; l'oxyde d'éthyle et de propynyle-2; l'oxyde d'éthynyle et de propyle; l'oxyde d'allyle et d'éthyle; l'oxyde d'allyle et d'isopropyle; l'oxyde d'isopropyle et de vinyle ou l'oxyde d'isobutyle et de vinyle;
– des éthers aliphatiques halogénés tels que l'oxyde de bromo-2 éthyle et d'éthyle; l'oxyde de chloro-2 éthyle et d'éthyle;
– des cétones aliphatiques telles que l'acétone; la butanone-2; la methyl-3 butanone-2; la diméthyl-3,3 butanone-2; la pentanone-2 ou la pentanone-3;
– des cétones aliphatiques chlorées que la chloro-3 butanone-2 ou la chloro-1 propanone-2.
7. Procédé selon l'une des revendications 1 à 6, caractérisé en ce que la quantité de solvant organique est telle que la concentration finale en acide salicylique dans ledit solvant soit au moins égale à 8% en poids.
8. Procédé selon l'une des revendications 1 à 7, caractérisé en ce que la température à laquelle il est mis en œuvre est comprise entre 10°C et la température d'ébullition du solvant organique et de préférence entre 40°C et 80°C.
9. Procédé selon l'une des revendications 1 à 8, caractérisé en ce que l'on utilise environ 105% à 110% de la quantité stoechiométrique d'acide sulfurique.

## Patentansprüche

1. Verfahren zur Reinigung von Salicylsäure aus einer wäßrigen Natriumsalicylat-Lösung, gekennzeichnet durch die folgende Aufeinanderfolge der Phasen:
1. Zugabe eines organischen Lösungsmittels für Salicylsäure zu der genannten wäßrigen Lösung in einer Menge, die ausreicht, um die dem eingesetzten Natriumsalz entsprechende Salicylsäure aufzulösen;
2. Zugabe einer starken Mineralsäure in einer Menge, welche mindestens der Stöchiometrie in bezug auf das Natriumsalicylat entspricht;
3. Abtrennung einer im wesentlichen organischen Phase, enthaltend die Salicylsäure, und einer wäßrigen Phase, enthaltend das gebildete Natriummineralsalz, und seine Behandlung in an sich bekannter Weise zur Abtrennung der Salicylsäure.
2. Verfahren gemäß Anspruch 1, dadurch gekennzeichnet, daß die verwendete starke Mineralsäure Schwefelsäure ist.
3. Verfahren gemäß einem der Ansprüche 1 oder 2, dadurch gekennzeichnet, daß das verwendete organische Lösungsmittel ausgewählt ist unter den aliphatischen Ethern, den substituierten, aliphatischen Ethern, den aliphatischen Ketonen, den halogenierten, aliphatischen Ketonen, den aliphatischen Aldehyden und den aliphatischen Alkoholen.
4. Verfahren gemäß einem der Ansprüche 1 bis 3, dadurch gekennzeichnet, daß das organische Lösungsmittel ausgewählt ist unter den aliphatischen Ethern, den chlorierten, aliphatischen Ethern, den aliphatischen Ketonen und den chlorierten, aliphatischen Ketonen.
5. Verfahren gemäß einem der Ansprüche 1 bis 4, dadurch gekennzeichnet, daß das organische Lösungsmittel einen Siedepunkt unterhalb oder gleich 120°C und vorzugsweise unterhalb oder gleich 100°C besitzt.
6. Verfahren gemäß einem der Ansprüche 1 bis 5, dadurch gekennzeichnet, daß das organische Lösungsmittel ausgewählt ist unter
– den aliphatischen Ethern, wie Diisopropylether, Methyl-tert.-butylether, Ethylisopropylether, Ethylpropylether, Butylethylether, Ethylisobutylether, Ethyl-tert.-butylether, Butylmethylether, Isobutylmethylether, Methylpentylether, Diethylether, Dipropylether, Isopropylpropylether, Ethyl-1-propinylether, Ethyl-2-propinylther, Ethinylpropylether, Allylethylether, Allylisopropylether, Isopropylvinylether oder Isobutylvinylether;

– den halogenierten, aliphatischen Ethern, wie 2-Bromethylethylether, 2-Chlorethylethylether;

– den aliphatischen Ketonen, wie Aceton, 2-Butanon, 3-Methyl-2-butanon, 3,3-Dimethyl-2-butanon, 2-Pentanon oder 3-Pentanon;

– den chlorierten, aliphatischen Ketonen, wie 3-Chlor-2-butanon oder 1-Chlor-2-propanon.

7. Verfahren gemäß einem der Ansprüche 1 bis 6, dadurch gekennzeichnet, daß die Menge an organischem Lösungsmittel derart ist, daß die Endkonzentration an Salicylsäure in dem genannten Lösungsmittel mindestens gleich 8 Gew.-% beträgt.

8. Verfahren gemäß einem der Ansprüche 1 bis 7, dadurch gekennzeichnet, daß die Temperatur, bei der gearbeitet wird, zwischen 10°C und der Siedetemperatur des organischen Lösungsmittels und vorzugsweise zwischen 40°C und 80°C liegt.

9. Verfahren gemäß einem der Ansprüche 1 bis 8, dadurch gekennzeichnet, daß man etwa 105 bis 110% der stöchiometrischen Menge an Schwefelsäure verwendet.

propyl ether; butyl ethyl ether; ethyl isobutyl ether; ethyl tert-butyl ether; butyl methyl ether; isobutyl methyl ether; methyl pentyl ether; diethyl ether; dipropyl ether; isopropyl propyl ether; ethyl 1-propynyl ether; ethyl 2-propynyl ether; ethynyl propyl ether; allyl ethyl ether; allyl isopropyl ether; isopropyl vinyl ether or isobutyl vinyl ether;

halogenated aliphatic ethers such as 2-bromoethyl ethyl ether; 2-chloroethyl ethyl ether;

aliphatic ketones such as acetone; 2-butanone; 3-methyl-2-butanone; 3,3-dimethyl-2 butanone; 2-pentanone or 3-pentanone;

chlorinated aliphatic ketones such as 3-chloro-2-butanone or 1-chloro-2-propanone;

7. Process according to one of claims 1 to 6, characterized in that the quantity of organic solvent is such that the final salicylic acid concentration in the said solvent is at least equal to 8% by weight.

8. Process according to one of claims 1 to 7, characterized in that the temperature at which it is carried out is between 10°C and the boiling point of the organic solvent and preferably between 40°C and 80°C.

9. Process according to one of claims 1 to 8, characterized in that approximately 105% to 110% of the stoichiometric quantity of sulphuric acid is used.

## Claims

1. Process for the purification of salicylic acid from an aqueous solution of sodium salicylate, characterized by the following sequence of stages:

1. addition to the said aqueous solution of an organic solvent of salicylic acid, in a quantity sufficient to dissolve the salicylic acid corresponding to the sodium salt employed;

2. addition of a strong inorganic acid in a quantity at least equal to the stoichiometry relative to the sodium salicylate.

3. separation of an essentially organic phase containing salicylic acid and of an aqueous phase containing the inorganic sodium salt formed and its treatment in a manner known per se to separate the salicyclic acid.

2. Process according to claim 1, characterized in that the strong mineral acid used is sulphuric acid.

3. Process according to one of claims 1 or 2, characterized in that the organic solvent used is chosen from aliphatic ethers, substituted aliphatic ethers, aliphatic ketones, halogenated aliphatic ketones, aliphatic aldehydes and aliphatic alcohols.

4. Process according to one of claims 1 to 3, characterized in that the organic solvent is chosen from aliphatic ethers, chlorinated aliphatic ethers, aliphatic ketones and chlorinated aliphatic ketones.

5. Process according to one of claims 1 to 4, characterized in that the organic solvent has a boiling point less than or equal to 120°C and preferably less than or equal to 100°C.

6. Process according to one of claims 1 to 5, characterized in that the organic solvent is chosen from: aliphatic ethers such as diisopropyl ether; methyl tert-butyl ether; ethyl isopropyl ether, ethyl